# EUROPEAN PATENT APPLICATION

(11) **EP 4 681 599 A1**
(43) Date of publication of application: **21.01.2026**
(21) Application number: 24188956.7
(22) Date of filing: 16.07.2024
(51) Int. Cl.: A61B 5/00, A61B 5/103, A61B 5/11, A61B 5/113, A61B 5/055

(54) **PRESSURE SENSOR MOTION DETECTION**

(71) Applicant: Koninklijke Philips N.V., 5656 AG Eindhoven (NL)
(72) Inventor: KRUEGER, Sascha, Eindhoven (NL); SENEGAS, Julien Thomas, Eindhoven (NL); VAN EE, Raymond, 5656AG Eindhoven (NL); WIEMKER, Rafael, Eindhoven (NL); WIRTZ, Daniel, Eindhoven (NL)
(74) Representative: Philips Intellectual Property & Standards

(57) **Abstract**

Disclosed herein is a medical system (100, 200, 400, 600) comprising a subject support (112) comprising a subject surface (114). The subject surface comprises an array (108) of spatially distributed pressure sensors (110). The medical system further comprises a remote spatial mapping system (106, 106') configured for measuring a spatial map (132) descriptive of objects (116, 902) on the subject surface. The execution of machine executable instructions (130) causes a computational system (120) to: receive (300) the spatial map (132); generate (302) a spatial object map (134) of objects placed on the subject support by inputting the spatial map into an object recognition module (136); determine (304) sensor specific signal scaling factors (138) using the spatial object map; repeatedly (306) receive the array of pressure data; and repeatedly (308) provide a subject motion signal (142) using the sensor specific signal scaling factors and the array of pressure data.

## Description

### FIELD OF THE INVENTION

The invention relates to medical imaging, in particular it relates to the detection and/or monitoring of subject motion during medical imaging procedures.

### BACKGROUND OF THE INVENTION

In various medical imaging modalities such as magnetic resonance imaging or computed tomography the subject may be inserted into a scanner. Cameras may be used to monitor various types of voluntary and involuntary subject motion. However, cameras are often times not able to image the complete subject during the imaging procedure.

International patent application WO2021256921A1 discloses a two-dimensional optical waveguide pressure sensor array is provided that comprises two or more row optical waveguides and two or more column optical waveguides. The row optical waveguides and the column optical waveguides are deformable and arranged in a planar array to define a sensor in the crosspoints. Each crosspoint includes one of the row waveguides in contact with one of the column waveguides at its intersection point. Each crosspoint further includes a light coupling structure configured to enhance waveguide bending when pressure is applied to the crosspoint. The light coupling structure comprises a layer of mechanical light scattering material disposed in contact with at least one of the row or column optical waveguide. The optical waveguide pressure sensor array is configured to sense pressure by providing light to the row optical waveguides and measuring light coupled at each crosspoint to its column optical waveguide, or vice versa, and wherein the light coupled to the column optical waveguide is dependent on the pressure applied to the crosspoint acting as a sensor.

### SUMMARY OF THE INVENTION

The invention provides for a medical system, a method, and a computer program product in the independent claims. Embodiments are given in the dependent claims.

In one aspect a medical system is disclosed. The medical system comprises a subject support comprising a subject surface configured for receiving a subject. The subject surface comprises an array of spatially distributed fiber optic pressure sensors configured for measuring an array of pressure data. The medical system further comprises a remote spatial mapping system configured for measuring a spatial map descriptive of objects on the subject surface. The remote spatial mapping system is configured to register the spatial map to the array of spatially distributed fiber optic pressure sensors. The medical system further comprises a memory storing machine-executable instructions.

In a preferred embodiment the array of spatially distributed pressure sensors is an array of fiber optic pressure sensors. In specific applications, e.g. in magnetic resonance imaging systems an array of fiber optic pressure sensors might be preferred as such an array might be manufactured using materials which do not, or to a limited extend, interact with the radiation field used in the imaging process. In other embodiments, other known pressure sensors might be applied.

The medical system further comprises a computational system. The execution of the machine-executable instructions causes the computational system to receive a spatial map from the remote spatial mapping system. Execution of the machine-executable instructions causes the computational system to generate a spatial object map of objects placed on the subject support by inputting the spatial map into an object recognition module. Execution of the machine-executable instructions further causes the computational system to determine sensor-specific signal scaling factors for the array of spatially distributed pressure sensors using the spatial object map. Execution of the machine-executable instructions further causes the computational system to repeatedly receive the array of pressure data from the array of spatially distributed pressure sensors. Execution of the machine-executable instructions further causes the computational system to repeatedly provide the subject motion signal by applying the sensor-specific signal scaling factors to the array of pressure data.

In another aspect, a method of operating a medical system is disclosed. The medical system comprises a subject support comprising a subject surface configured for receiving a subject. The subject surface comprises an array of spatially distributed pressure sensors configured for measuring an array of pressure data. The medical system further comprises a remote spatial mapping system configured for measuring a spatial map descriptive of objects on the subject surface. The remote spatial mapping system is configured to register the spatial map to the array of spatially distributed pressure sensors.

The method comprises receiving a spatial map from the remote spatial mapping system. The method further comprises generating a spatial object map of objects placed on the subject support by inputting the spatial map into an object recognition module. The method further comprises determining the sensor-specific signal scaling factors for the array of spatially distributed pressure sensors using the spatial object map. The method further comprises repeatedly receiving the array of pressure data from the array of spatially distributed pressure sensors. The method further comprises repeatedly providing a subject motion signal by applying the sensor-specific signal scaling factors to the array of pressure data.

Again, in a preferred embodiment the array of spatially distributed pressure sensors is an array of fiber optic pressure sensors. In specific applications, e.g. in magnetic resonance imaging systems an array of fiber optic pressure sensors might be preferred as such an array might be manufactured using materials which do not, or to a limited extend, interact with the radiation field used in the imaging process. In other embodiments, other known pressure sensors might be applied.

In another aspect a computer program product comprising machine-executable instructions is disclosed. Execution of the machine-executable instructions causes a computational system to perform a method as disclosed above.

### BRIEF DESCRIPTION OF THE DRAWINGS

In the following preferred embodiments of the invention will be described, by way of example only, and with reference to the drawings in which:
Fig. 1 illustrates an example of a medical system.
Fig. 2 illustrates a further example of a medical system.
Fig. 3 shows a flow chart which illustrates a method of using the medical system of Fig. 1 or of Fig. 2.
Fig. 4 illustrates a further example of a medical system.
Fig. 5 shows a flow chart which illustrates a method of using the medical system of Fig. 4.
Fig. 6 illustrates a further example of a medical system.
Fig. 7 illustrates an example of a subject support with an array of spatially distributed fiber optic pressure sensors.
Fig. 8 shows a further view of the subject support of Fig. 7.
Fig. 9 shows an example of a spatial map overlaid with a spatial object map.

### DETAILED DESCRIPTION OF EMBODIMENTS

Like numbered elements in these figures are either equivalent elements or perform the same function. Elements which have been discussed previously will not necessarily be discussed in later figures if the function is equivalent.

In an example, there is a medical system. The medical system comprises a subject support comprising a subject surface configured for receiving a subject. The subject surface may for example be configured for receiving an entire subject, in which case the subject would lay down on the subject support, or it may be a subject support configured for receiving a part or portion of the subject such as a limb or a head region. The subject surface comprises an array of spatially distributed fiber optic pressure sensors configured for measuring an array of pressure data. Fiber optic pressure sensors as used herein encompass pressure sensors which operate by the transmission of light through optical fibers and the interaction of light with the pressure sensor. Various types of fiber optic pressures sensors may be used such as intensity-based sensors, interferometer -based sensors, and Fabry-Perot sensors. The advantage of using fiber optic pressure sensors is that they are compatible with several different modes of medical imaging or tomographic medical imaging in particular. For example, the fiber optic pressure sensors are compatible with magnetic resonance imaging systems and with computed tomography systems. The array of pressure data may for example represent the value of pressure measured by an individual fiber optic pressure sensor at a particular time or interval of time.

The medical system further comprises a remote spatial mapping system that is configured for measuring a spatial map that is descriptive of objects on the subject surface. The remote spatial mapping system is configured to register the spatial map to the array of spatially distributed fiber optic pressure sensors. As will be described below, the remote spatial mapping system may be implemented in different way such as using imaging systems as cameras, infra-red cameras, 3D cameras as well as other systems such as ultrasound detection and radar. The spatial map provides information on the distribution of objects on the subject surface.

The medical system further comprises a memory storing machine-executable instructions. The medical system further comprises a computational system. Execution of the machine-executable instructions causes the computational system to receive a spatial map from the remote spatial mapping system. For example, if the remote spatial mapping system is a camera, the spatial map would be an image that is registered to the array of spatially distributed fiber optic pressure sensors. Execution of the machine-executable instructions further causes the computational system to generate a spatial object map of objects placed on the subject support by inputting the spatial map into an object recognition module. The spatial object map may take different forms depending upon the type of data in the spatial map. If the spatial map is for example an image the spatial object map may identify objects as well as the position or anatomical position of a subject on the subject surface.

Execution of the machine-executable instructions further causes the computational system to determine sensor-specific signal scaling factors for the array of spatially distributed fiber optic pressure sensors using the spatial object map. The spatial object map identifies the location of objects on the surface and is registered to the location of the spatially distributed fiber optic pressure sensors. A knowledge of which objects are over or adjacent to which particular sensor can be used to provide a scaling factor for individual fiber optic pressure sensors. For example, if there is a heavy object such as a head support which is placed on the subject support, it may be desirable to disregard the pressure sensors which are affected by this particular object. Likewise, other medical devices or appliances may be placed on the subject surface along with the subject. The sensor-specific scaling factors could be used to essentially turn off the signal from a particular sensor or to provide a scaling to account for the magnitude in different sensor signals having different baseline values.

Execution of the machine-executable instructions further causes the computational system to repeatedly receive the array of pressure data from the array of spatially distributed fiber optic pressure sensors. For example, the subject could be inserted into a medical imaging system and while the subject is being imaged using the medical imaging system the array of pressure data is repeatedly received. Execution of the machine-executable instructions further causes the computational system to repeatedly provide a subject motion signal by applying the sensor-specific signal scaling factors to the array of pressure data. The array of pressure data is received and then it can be modified by applying the sensor-specific scaling factors to the received data and this can be done and then used to provide the subject motion signal. The sensor-specific scaling factors may take different forms in different examples. In one example, the scaling factors are used to attenuate or amplify the contribution of pressure data from individual pressor sensors. In other cases, the sensor-specific scaling factors may be used to adjust the contribution of individual pressor sensors to a linear or non-linear model used to produce the subject motion signal.

In some examples, the subject motion signal may comprise more than one value or signals. The one or more values may be constructed using linear or non-linear combinations of the array of pressure data. The sensor specific signal scaling factors may be used to scale individual values from individual sensors or may be used to construct linear combinations of values from multiple sensors. This example may be beneficial because it may provide for an accurate means of measuring subject motion within a medical imaging system even when it is not possible to monitor body parts or portions of a subject which are moving and may affect image quality during the medical imaging procedure.

In another aspect execution of the machine-executable instructions further causes the computational system to calibrate the subject motion signal by repeatedly receiving the array of pressure data from the array of spatially distributed fiber optic pressure sensors. Execution of the machine-executable instructions further causes the computational system to calibrate the subject motion signal by receiving sensor data descriptive of the subject motion during the acquisition of the repeatedly received array of pressure data. The sensor data may be used to describe a particular type of voluntary or involuntary motion of the subject. Execution of the machine-executable instructions further causes the computational system to calibrate the subject motion signal by determining a correlation between the sensor data and the repeatedly received array of pressure data. The subject motion signal is then at least partially constructed using the correlation. For example, the correlation may be a mapping between the sensor data and particular values or changes in the pressure data. This means that if the correlation or mapping is known, then when the pressure data is measured, this may be used to for example reproduce or provide an artificial subject motion which is comparable or provides similar data to the sensor data. This may for example be beneficial because it may provide for a means of providing a subject motion signal which is descriptive of motion of the subject in the same way that the sensor data is. This may for example enable the detection of the subject motion without the particular sensor for the sensor data being present during the imaging procedure.

In another example, the sensor data comprises the spatial map. For example, in images and three-dimensional images it is possible to detect such things as changes of color or movement which can be used to predict such things as heart and breathing movement. Additionally, the spatial map may be able to track and respond to motion of the subject's body such as moving limbs, moving the head or even moving fingers. In this respect, the sensor data can contain information from the spatial map directly in some examples.

In another example, the medical system further comprises an external sensor system configured for acquiring the sensor data. For example, there may be heart monitors or breathing monitors which are connected to the subject during a calibration phase, which may not be practical or possible to insert into the medical imaging system during a medical imaging procedure. This may include such things as putting wired ECG sensors on the subject, wearing a breathing belt or other respiration measuring device or other sensor system.

In another example, the correlation is determined using any one of the following: a principle component analysis, a neural network, a statistical model, and combinations thereof. For example, principle component analysis can be used to provide very accurate mappings between the two. A neural network may for example be a neural network that contains multiple fully connected layers or convolutional layers and may be trained to receive as input the array of pressure data and the sensor data and then output correlations. Various statistical models such as determining correlation coefficients could also be used.

In another example the subject motion signal is any one of the following: a respiratory phase signal, a cardiac phase signal, a finger motion signal, and combinations thereof. It actually may be possible to measure or provide multiple subject motion signals depending upon the particular type of motion. For example, the respiratory phase signal may have a longer period and different pressure sensors which are activated then for the cardiac phase signal or the finger motion signal. The finger motion signal may be particularly beneficial for magnetic resonance imaging systems as when the subject touches him or herself, she may form a ground loop which may then lead to heating or the formation of a current in this ground loop. Often times in a magnetic resonance imaging system it is not possible to image the entire subject. This may be beneficial because it may provide a means of detecting potential motion which is currently not possible.

In another example, the object recognition module is a trained object recognition module. An object, as used herein, may encompass the subject, other personnel, or devices or appliances or objects which may be placed on the subject surface. In some instances, the trained object recognition neural network may be a collection of neural networks which are used together. For example, the trained object recognition neural network may comprise a neural network which is trained to recognize various anatomical key points. This may provide a detailed information on the position of a subject on the subject support. In other cases, one of these neural networks may provide for a convolutional neural network which is able to output bounding boxes which identify the location of various objects or appliances which may also be placed on the subject surface. Both the identification of anatomical key points as well as the identification of the location of objects are possible using currently available neural networks. The combination of the two may provide a flexible means of identifying both subjects and inanimate or animate objects placed on the subject support.

In another example, the spatial object map comprises individual bounding boxes and item identifiers for the individual bounding boxes. The sensor-specific signal scaling factor is assigned according to the item identifiers for the individual bounding boxes for pressure sensors within a predetermined distance to the individual bounding boxes. The bounding boxes may identify what object and where it is placed relative to the various pressure sensors. Depending upon if a pressure sensor is within the bounding box or within a predetermined distance, then this can be used to directly assign the sensor-specific scaling factor. For example, a bounding box may identify a spatial location and then provide an item identifier. This can be used for example with a lookup table or database to provide the sensor-specific scaling factor for the fiber optic pressure sensors that are affected. The neural network in this aspect, as was mentioned above, may be provided by a convolutional neural network that is configured for identifying objects within images. This may for example be trained by having images which have been manually labeled with identifiers and objects on a particular subject support. These labeled images then may be used for example in a deep learning routine to train the neural network.

In another example, the spatial object map comprises individual anatomical key points. The sensor-specific signal scaling factor is assigned according to individual anatomical key points for pressure sensors within a predetermined distance to the individual anatomical key points. Various convolutional neural networks are currently available which are able to provide or identify anatomical key points which may for example identify the location of joints or particular organs or portions of a subject directly. This may then provide information on which anatomical parts of the subject are directly located or adjacent to a particular fiber optic pressure sensor. A knowledge of this proximity and also the scaling factor can be used to then assign the scaling factors to the particular fiber optic pressure sensors. This may for example be particularly beneficial when there is also a knowledge of a particular medical imaging protocol which is being used. For example, if a medical imaging protocol involves imaging the head, then various portions of the subject which are not adjacent to, near or motion-wise do not correlate with the head may for example be disregarded. Likewise, the anatomical key points can be used to identify regions of the distributed fiber optic pressure sensors which may be relevant to cardiac and/or respiratory monitoring.

In another example, the remote spatial mapping system is any one of the following: an optical camera, an infra-red camera, an optical remote spatial mapping system, and a three-dimensional camera. In this example each of these three examples provide a means of providing image data which can for example be processed using a neural network to identify objects on the subject surface.

In another example, the remote spatial mapping system is a radar system or an ultrasound system. The use of a radar system or an ultrasound system may for example provide an alternative means of providing the spatial object map.

In another example, the medical system further comprises a medical imaging system. Execution of the machine-executable instructions further causes the computational system to control the medical imaging system to acquire the medical image data descriptive of the subject and then to adjust the acquisition of the medical imaging system during the acquisition of the medical image data using the subject motion signal. This may for example provide for a better control of the medical imaging system and/or provide for better detection of subject motion. This may for example lead to improved quality of imaging the subject. In another example, execution of the machine-executable instructions further causes the computational system to reconstruct a medical image from the medical image data.

In another example, the medical system is a magnetic resonance imaging system or a computed tomography system. Both examples may be beneficial because in many cases the imaging modalities are not compatible with all types of detectors of subject motion. For example, a respiratory belt or other sensors may interfere with the proper operation of a magnetic resonance imaging system and/or a computed tomography system. The integration of the subject support into the medical imaging system may enable the detection of subject motion signals either more accurately or also in cases where it would not be possible to detect such things such as finger motion.

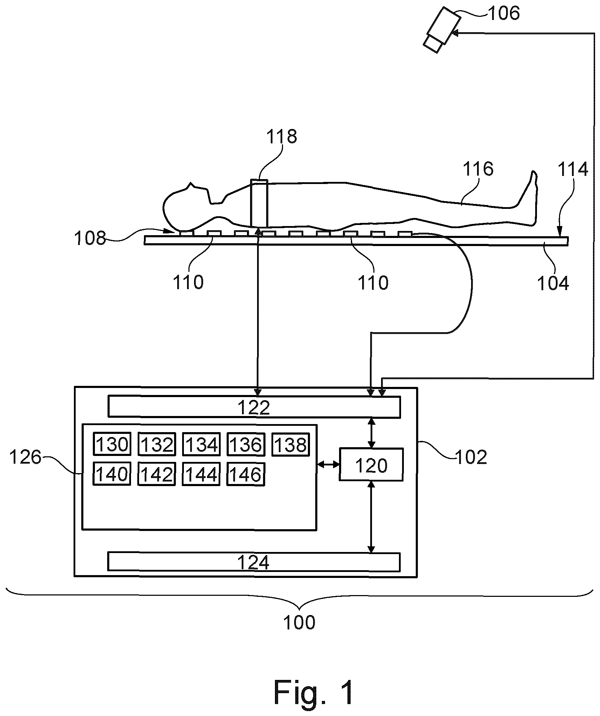

Fig. 1 illustrates an example of a medical system 100. This medical system 100 is shown as comprising a computer 102 that is connected to a subject support 104 and a camera system 106. The camera system 106 functions as a remote spatial mapping system in this example. An array of spatially distributed fiber optic pressure sensors 108 are distributed on a subject surface 114 of a subject support 112. Several individual fiber optic pressure sensors 110 are individually marked. A subject 116 is reposing on the subject support 104. The subject 116 is also shown as wearing an optional external sensor system 118. In this particular example, the external sensor system 118 is a respiration belt.

The computer 102 is shown as comprising a computational system 120 that is connected to a hardware interface 122 and an optional user interface 124. The computational system 120 is intended to represent one or more computational systems or computers located at one or more locations. The hardware interface 122 enables the computational system 120 to communicate and control the other components of the medical system 100. The user interface 124, if it is present, may be used to operate and control the medical system 100.

The computational system 120 is also shown as being connected to a memory 126. The memory 126 is intended to represent various types of memories which may be connected or accessible to the computational system 120.

The memory 126 is shown as storing machine-executable instructions 130. The machine-executable instructions 130 enable the computational system 120 to control and operate the medical system 100 as well as perform various image processing and numerical tasks. The memory 126 is further shown as containing a spatial map 132 that was acquired by making an image with the camera system 106. The camera system 106 could for example be replaced with an infra-red camera or a three-dimensional camera. The memory 126 is further shown as containing a spatial object map 134 that was obtained by inputting the spatial map 132 into an object recognition module 136. The spatial object map 134 may for example contain anatomical key points of the subject 116 as well as the location of other objects that are placed on the subject surface 114. The spatial object map 134 has its locations registered to the location of the individual fiber optic pressure sensors 110.

Using the object recognition module 136 sensor-specific scaling factors 138 for the array of spatially distributed fiber optic pressure sensors 108 is determined. The sensor-specific scaling factors 138 may for example be dependent on a chosen medical imaging protocol to look for particular types of motion by the subject 116. The memory 126 is further shown as containing a repeatedly acquired array of pressure data 140 acquired by the individual fiber optic pressure sensors 110. The memory 126 is further shown as containing a subject motion signal 142 that was constructed using the repeatedly acquired array of pressure data 140 and the sensor-specific scaling factors 138. The memory 126 is further shown as containing optional sensor data 144 acquired from the external sensor system 118. The memory 126 is further shown as containing an optional correlation between the sensor data and the repeatedly received array of pressure data 140. For example, this correlation could be used to provide a subject motion signal 142 that is equivalent or maps the sensor data 144. For example, this may enable the replacement of the respiratory belt 118 with the use of the array of spatially distributed fiber optic pressure sensors 108. In other examples, other types of external sensor systems 118 may be used. In some examples the camera system 106 may also provide the sensor data. For example, if the camera system 106 is a color camera it may be able to note the change in the appearance of the subject's exposed skin on the face to measure the heart phase.

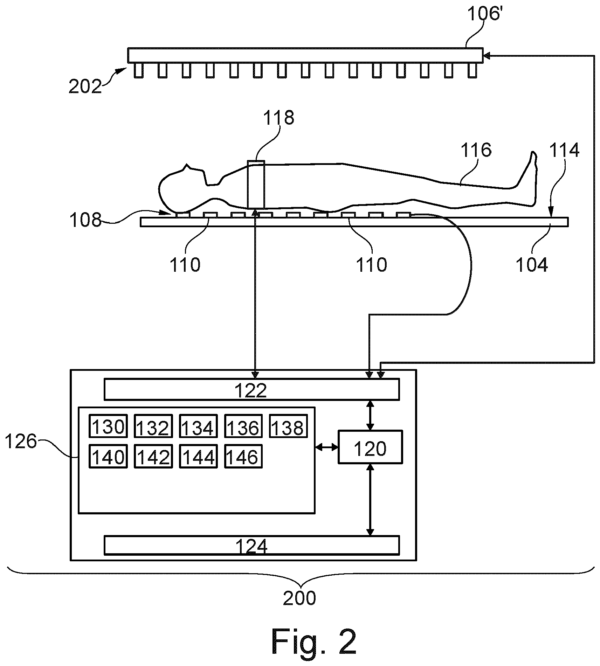

Fig. 2 illustrates a further example of a medical system 200. The medical system 200 illustrated in Fig. 2 is similar to that depicted in Fig. 1 except the camera system 106 has been replaced with a radar array or ultrasound array 106'. There are a number of transceiver elements 202 that are positioned in an array above the subject 116. The use of the remote spatial mapping system 106' provides an alternative to using an optical system as is illustrated in Fig. 1.

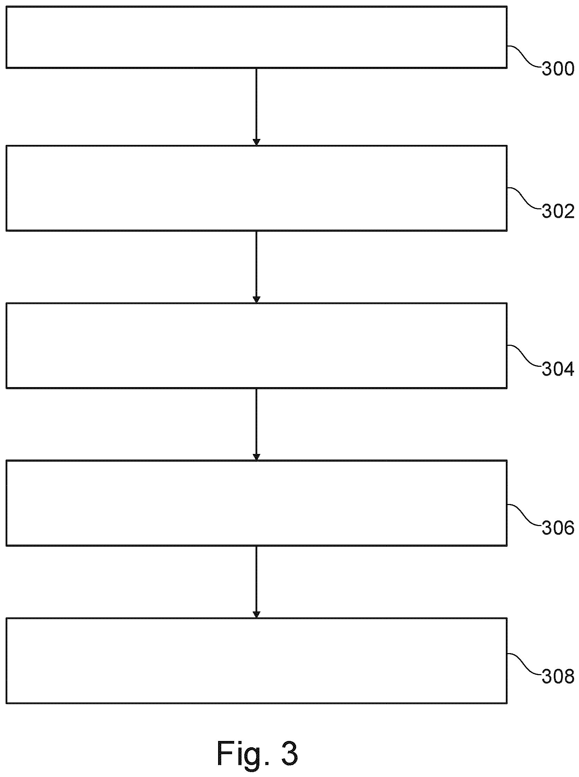

Fig. 3 shows a flowchart which illustrates a method of operating the medical system 100 of Fig. 1 or the medical system 200 of Fig. 2. In step 300, the spatial map 132 is received from the remote spatial mapping system 106 or 106'. In step 302, a spatial object map 134 of objects 116 placed on the subject support 104 is generated by inputting the spatial map 132 into the object recognition module 136. In step 304, the sensor-specific scaling factors 138 for the array of spatially distributed fiber optic pressure sensors 108 is determined using the spatial object map 134. In step 306, the array of pressure data 140 is repeatedly received from the array of spatially distributed fiber optic pressure sensors 108. In step 308, the subject motion signal 142 is provided repeatedly by applying the sensor-specific scaling factors 138 to the array of pressure data 140.

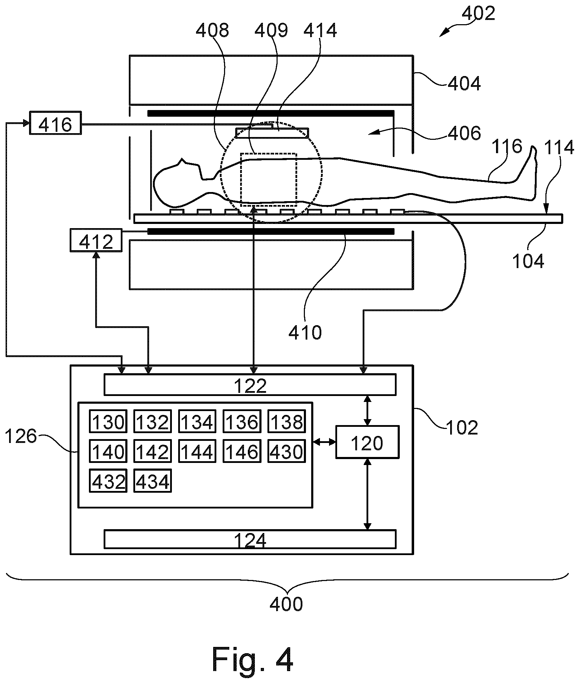

Fig. 4 illustrates a further example of a medical system 400. In this example the medical system 400 comprises the magnetic resonance imaging system 402 along with the subject support 104. The camera system 106 as illustrated in Fig. 1 or the radar array or ultrasound array 106' as illustrated in Fig. 2 could be incorporated outside of the magnetic resonance imaging system 402 or at a different location. For example, in the first case, the remote spatial mapping system 106, 106' could be immediately outside the bore of the magnetic resonance imaging system 402 and then the subject support 104 along with the subject 106 is injected into the bore 406. In other examples the subject support 104 may be removable from the magnetic resonance imaging system 402 and the camera system 106 or the radar array or ultrasound array 106' could be at a different location. The features of Figs. 1 and/or 2 may be combined with the features of Fig. 4.

The magnetic resonance imaging system 402 comprises a magnet 404. The magnet 404 is a superconducting cylindrical type magnet with a bore 406 through it. It is also possible to use both a split cylindrical magnet and a so-called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject. The arrangement of the two sections area is similar to that of a Helmholtz coil. Open magnets are popular because the subject is less confined. Inside the cryostat of the cylindrical magnet there is a collection of superconducting coils.

Within the bore 406 of the cylindrical magnet 404 there is an imaging zone 408 where the magnetic field is strong and uniform enough to perform magnetic resonance imaging. A field of view 309 is shown within the imaging zone 308. The k-space data are acquired for the field of view 409. The region of interest could be identical with the field of view 409 or it could be a sub volume of the field of view 409. A subject 116 is shown as being supported by the subject support 104 such that at least a portion of the subject 116 is within the imaging zone 408 and the field of view 409.

Within the bore 406 of the magnet there is also a set of magnetic field gradient coils 410 which is used for the acquisition of measured k-space data to spatially encode magnetic spins within the imaging zone 408 of the magnet 404. The magnetic field gradient coils 410 are connected to a magnetic field gradient coil power supply 412. The magnetic field gradient coils 410 are intended to be representative. Typically, magnetic field gradient coils 410 contain three separate sets of coils for spatial encoding in three orthogonal spatial directions. A magnetic field gradient power supply supplies current to the magnetic field gradient coils. The current supplied to the magnetic field gradient coils 410 is controlled as a function of time and may be ramped or pulsed.

Adjacent to the imaging zone 408 is a radio frequency coil 414 for manipulating the orientations of magnetic spins within the imaging zone 408 and for receiving radio transmissions from spins also within the imaging zone 408. The radio frequency antenna may contain multiple coil elements. The radio frequency antenna may also be referred to as a channel or antenna. The radio frequency coil 414 is connected to a radio frequency transceiver 416. The radio frequency coil 414 and radio frequency transceiver 416 may be replaced by separate transmit and receive coils and a separate transmitter and receiver. It is understood that the radio frequency coil 414 and the radio frequency transceiver 416 are representative. The radio frequency coil 414 is intended to also represent a dedicated transmit antenna and a dedicated receive antenna. Likewise, the transceiver 416 may also represent a separate transmitter and receiver. The radio frequency coil 414 may also have multiple receive/transmit elements and the radio frequency transceiver 416 may have multiple receive/transmit channels. The transceiver 416 and the gradient controller 412 are shown as being connected to the hardware interface 122 of the computer system 102.

The magnetic resonance imaging system 402 is shown as being controlled by the computer system 402. The memory 126 is further shown as containing pulse sequence commands 430. The pulse sequence commands are commands or data which may be converted into commands to control the magnetic resonance imaging system 402 for a particular magnetic resonance imaging protocol. The memory 126 is further shown as containing k-space data 432 that was acquired by controlling the magnetic resonance imaging system 402 with the pulse sequence commands 430. The memory 126 is also shown as containing an optional magnetic resonance image 434 that was reconstructed from the k-space data 432.

The subject motion signal 142 may be used in different ways when controlling the magnetic resonance imaging system with the pulse sequence commands 430. For example, the subject motion signal 142 could be used to trigger or engage the acquisition of the k-space data 432. In other instances it may be used for example to cancel or restart the acquisition of the k-space data 432 if it is determined that the subject 116 has moved too much.

It should also be noted that the use of a magnetic resonance imaging system 402 in Fig. 4 and the following Figs. is only an example. The magnetic resonance imaging system 402 may for example also be replaced with a computed tomography system.

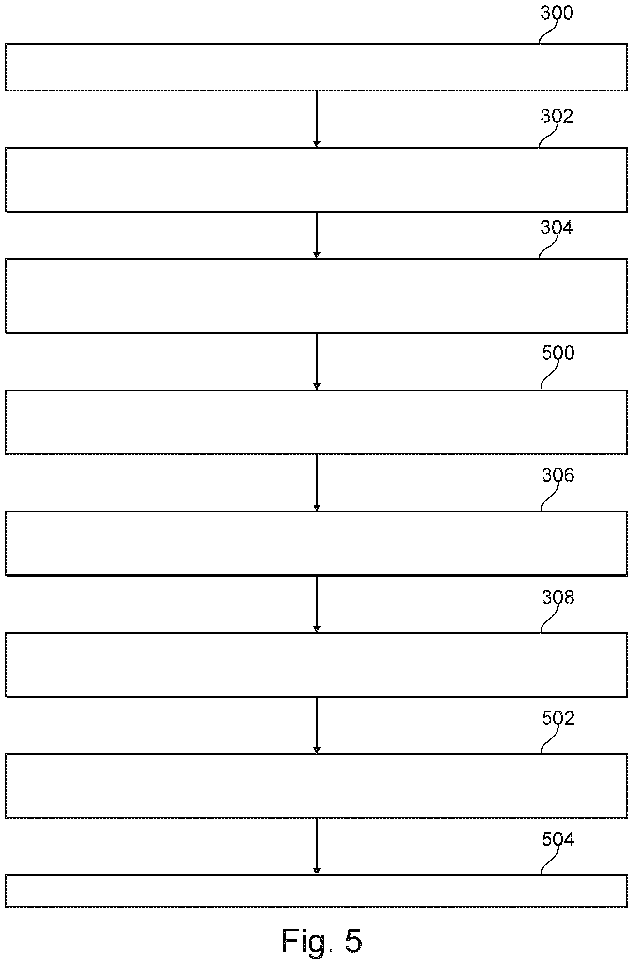

Fig. 5 shows a flowchart which illustrates a method of operating the medical system 400 of Fig. 4. The method starts with steps 300, 302, 304 as was illustrated in Fig. 3. In step 500, the medical imaging system 402 is controlled to acquire the medical image data 432. In this case the medical imaging system is a magnetic resonance imaging system 402 and the medical image data is the k-space data 432. In the case of a computed tomography system the medical image data would be X-ray profiles. Steps 306 and 308 are then performed, as was illustrated in Fig. 3. In step 502 the medical imaging system 402 is adjusted during the acquisition of the medical image data 432 using the subject motion signal 142. Step 504 is optional. In step 504 the medical image 434 is reconstructed from the medical image data 432. In some instances, the medical image data 432 may be sent to a remote or cloud-based server to perform the image reconstruction.

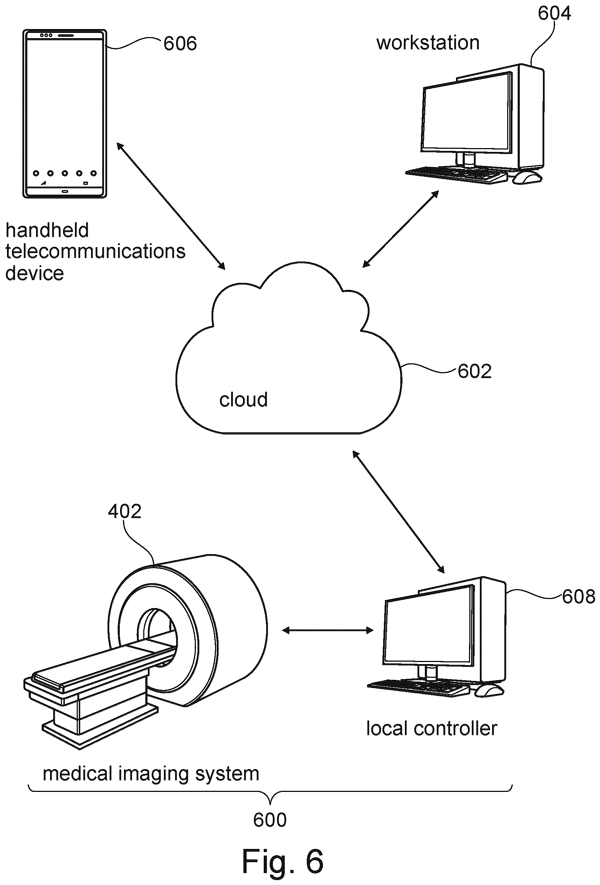

Fig. 6 illustrates a further example of a medical system 600. The medical system 600 comprises the medical imaging system (i.e., the magnetic resonance imaging system 402 of Fig. 4) as well as a cloud based computing system 602, a radiology workstation 604, a handheld telecommunications device 606, and a local controller 608. The functionality of the computer 102 depicted in Figs. 1, 2, and 4 can be distributed amongst the various devices 702, 704, 706, and 708. The cloud based computing system 702 could be made up of networked servers and/or virtual machines available over the internet or other network.

A typical configuration would be to use the local controller 708 to use the pulse sequence commands 430 to control the magnetic resonance imaging system 402 to acquire the k-space data 432. The reconstruction of the initial magnetic resonance image as well as the clinical magnetic resonance image may be performed by the cloud based computing system 702. The resulting magnetic resonance image 434 would then be made available to the workstation 704, the handheld telecommunications device 706, and the local controller 708.

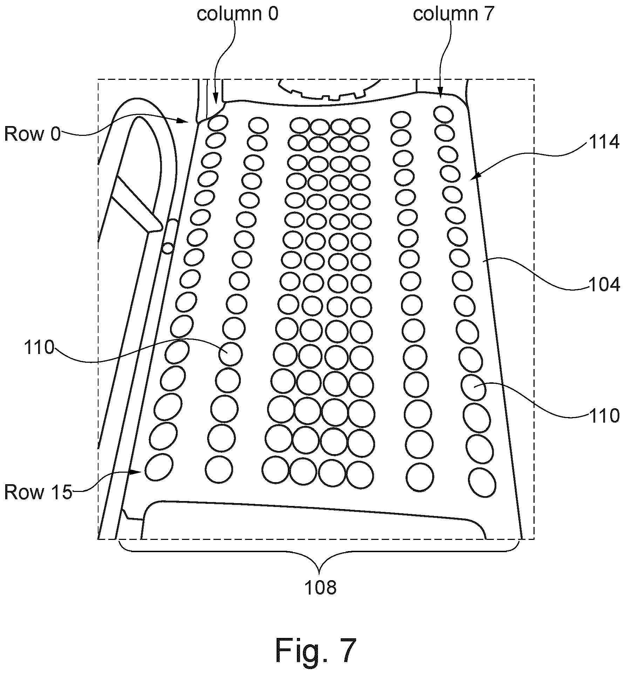

Fig. 7 illustrates an example of a subject support 104 that has an array of spatially distributed fiber optic pressure sensors 108 on the subject surface 114. In this example the arrays have 8 columns and 16 rows and is sufficient to provide sensors for the upper torso of a subject. The distribution of sensors 110 is sufficient for monitoring such things as breathing, heart signal, and also fine body movement such as finger movement.

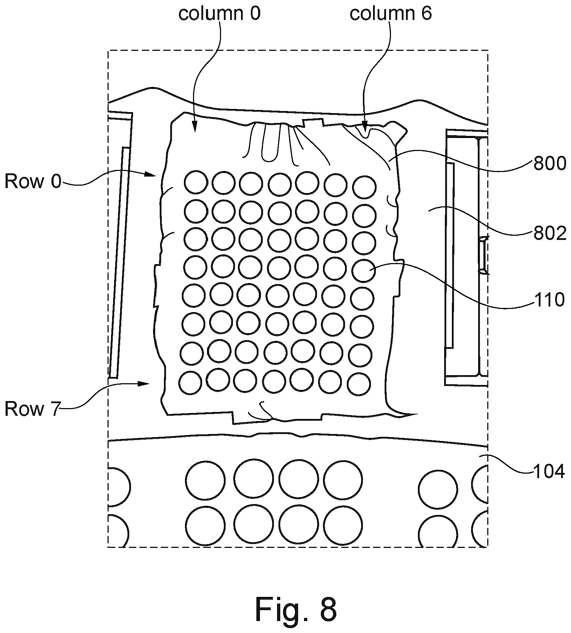

Fig. 8 shows a further view of the subject support 104 depicted in Fig. 7. There is additionally a view of a head support 802 with additional sensors 800 that are added to the array. In this example the sensors are distributed to measure the torso in Fig. 7 and additionally there are sensors to measure the movement of the subject's head. As is illustrated in Figs. 7 and 8, it is straightforward to modify existing subject supports and be able to monitor either the torso or extremities of the subject effectively.

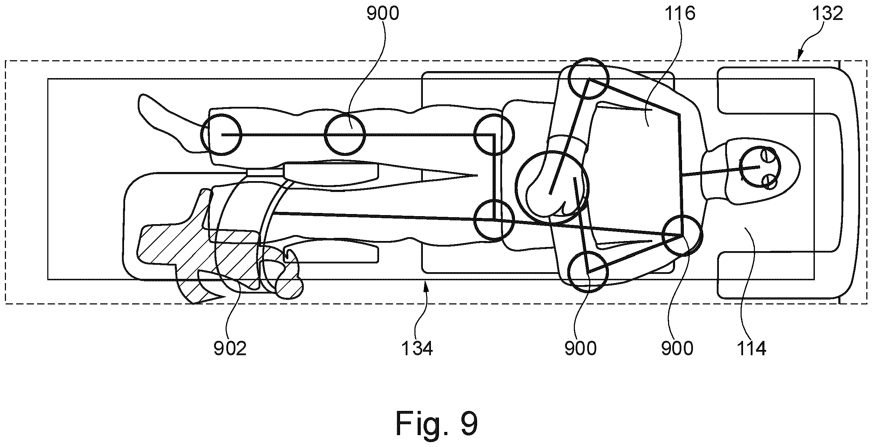

Fig. 9 shows a view of a spatial object map 134 superimposed on a spatial map 132. In this case the spatial map 132 is an image and the spatial object map 134 contains anatomical key points 900 as well as additional objects 902. A subject 116 is shown as reposing on the subject support 104 illustrated in Fig. 7 and 8. It can be seen that the hands of the subject 116 have some of the fingers obscured. A camera would not be able to detect all finger motion in this case, however the pressure sensors 108 would be able to.

It is understood that one or more of the aforementioned examples or embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

`Computer memory' or 'memory' is an example of a computer-readable storage medium. Computer memory is any memory which is directly accessible to a computational system. `Computer storage' or 'storage' is a further example of a computer-readable storage medium. Computer storage is any non-volatile computer-readable storage medium. In some embodiments computer storage may also be computer memory or vice versa.

A `computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A `user interface' as used herein is an interface which allows a user or operator to interact with a computer or computer system. A `user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A `hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or `display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: medical system
- 102: computer
- 104: subject support
- 106: camera system (remote spatial mapping system)
- 106': radar array or ultrasound array
- 108: array of spatially distributed fiber optic pressure sensors
- 110: individual fiber optic pressure sensor
- 112: subject support
- 114: subject surface
- 116: subject
- 118: optional external sensor system
- 120: computational system
- 122: hardware interface
- 124: optional user interface
- 126: memory
- 130: machine executable instructions
- 132: spatial map
- 134: spatial object map
- 136: object recognition module
- 138: sensor specific scaling factors
- 140: repeatedly acquired array of pressure data
- 142: subject motion signal
- 144: optional sensor data
- 146: optional correlation between sensor data and the repeatedly received array of pressure data
- 200: medical system
- 202: transceiver element
- 300: receive a spatial map from the remote spatial mapping system
- 302: generate a spatial object map of objects placed on the subject support by inputting the spatial map into an object recognition module
- 304: determine sensor specific signal scaling factors for the array of spatially distributed fiber optic pressure sensors using the spatial object map
- 306: repeatedly receive the array of pressure data from the array of spatially distributed fiber optic pressure sensors
- 308: repeatedly provide a subject motion signal by applying the sensor specific signal scaling factors to the array of pressure data
- 400: medical system
- 402: magnetic resonance imaging system
- 404: magnet
- 406: bore of magnet
- 408: imaging zone
- 409: field of view
- 410: magnetic field gradient coils
- 412: magnetic field gradient coil power supply
- 414: radio frequency coil
- 416: transceiver
- 430: pulse sequence commands
- 432: k-space data (medical image data)
- 434: magnetic resonance image
- 500: controlling the medical imaging system to acquire medical image data descriptive of the subject
- 502: adjust the medical imaging system during the acquisition of medical image data using the subject motion signal
- 600: medical system
- 602: cloud computing system
- 604: radiology workstation
- 606: handheld telecommunication device
- 608: local controller
- 800: additional sensors for array
- 802: head support
- 900: anatomical keypoints
- 902: additional objects

## Claims

1. A medical system (100, 200, 400, 600) comprising:
- a subject support (112) comprising a subject surface (114) configured for receiving a subject (116), wherein the subject surface comprises an array (108) of spatially distributed pressure sensors (110), preferably fiber optic pressure sensors, configured for measuring an array of pressure data;
- a remote spatial mapping system (106, 106') configured for measuring a spatial map (132) descriptive of objects (116, 902) on the subject surface, wherein the remote spatial mapping system is configured to register the spatial map to the array of spatially distributed pressure sensors;
- a memory (126) storing machine executable instructions (130);
- a computational system (120), wherein execution of the machine executable instructions causes the computational system to:
- receive (300) the spatial map (132) from the remote spatial mapping system;
- generate (302) a spatial object map (134) of objects placed on the subject support by inputting the spatial map into an object recognition module (136);
- determine (304) sensor specific signal scaling factors (138) for the array of spatially distributed pressure sensors using the spatial object map;
- repeatedly (306) receive the array of pressure data from the array of spatially distributed pressure sensors; and
- repeatedly (308) provide a subject motion signal (142) by applying the sensor specific signal scaling factors to the array of pressure data.

2. The medical system of claim 1, wherein execution of the machine executable instructions further causes the computational system to calibrate the subject motion signal by:
- repeatedly receiving the array of pressure data from the array of spatially distributed pressure sensors;
- receiving sensor data (144) descriptive of the subject motion during the acquisition of the repeatedly received array of pressure data; and
- determining a correlation (146) between the sensor data and the repeatedly received array of pressure data; and wherein the subject motion signal is at least partially constructed using the correlation.

3. The medical system of claim 2, wherein the sensor data comprises the spatial map.

4. The medical system of claim 2 or 3, wherein the medical system further comprises an external sensor system (118) configured for acquiring the sensor data.

5. The medical system of claim 2, 3, or 4, wherein the correlation is determined using any one of the following: principal component analysis, a neural network, a statistical model, and combinations thereof.

6. The medical system of any one of the preceding claims, wherein the subject motion signal is any one of the following: a respiratory phase signal, a cardiac phase signal, a finger motion signal, and combinations thereof.

7. The medical system of any one of the preceding claims, wherein the object recognition module is a trained object recognition neural network.

8. The medical system of claim 7, wherein the spatial object map comprises individual anatomical key points (900), wherein the sensor specific signal scaling factor is assigned according to individual anatomical key points for pressure sensors within a predetermined distance to the individual anatomical key points.

9. The medical system of claim 7 or 8, wherein the spatial object map comprises individual bounding boxes and item identifiers for the individual bounding boxes, wherein the sensor specific signal scaling factor is assigned according to the item identifiers for the individual bounding boxes for pressure sensors within a predetermined distance to the individual bounding boxes.

10. The medical system of any one of the preceding claims, wherein the remote spatial mapping system (106) is any one of the following, an optical camera, an infrared camera, an optical remote spatial mapping system, and a three-dimensional camera.

11. The medical system of any one of claims 1 through 9, wherein the remote spatial mapping system (106') is a radar system or an ultrasound system.

12. The medical system of any one of the preceding claims, wherein the medical system further comprises a medical imaging system (402), wherein execution of the machine executable instructions further causes the computational system to:
- controlling (500) the medical imaging system to acquire medical image data (432) descriptive of the subject; and
- adjust (502) the medical imaging system during the acquisition of medical image data using the subject motion signal.

13. The medical system of claim 12, wherein the medical system is a magnetic resonance imaging system (402) or a computed tomography system.

14. A method of operating a medical system (100, 200, 400, 600), wherein the medical system comprises a subject support (112) comprising a subject surface (114) configured for receiving a subject (116), wherein the subject surface comprises an array (108) of spatially distributed pressure sensors (110), preferably fiber optic pressure sensors, configured for measuring an array of pressure data, wherein the medical system further comprises a remote spatial mapping system (106, 106') configured for measuring a spatial map (132) descriptive of objects (116, 902) on the subject surface, wherein the remote spatial mapping system is configured to register the spatial map to the array of spatially distributed pressure sensors, wherein the method comprises:
- receiving (300) the spatial map from the remote spatial mapping system;
- generating (302) a spatial object map (134) of objects placed on the subject support by inputting the spatial map into an object recognition module (136);
- determining (304) sensor specific signal scaling factors (138) for the array of spatially distributed pressure sensors using the spatial object map;
- repeatedly (306) receiving the array of pressure data from the array of spatially distributed pressure sensors; and
- repeatedly (308) providing a subject motion signal (142) by applying the sensor specific signal scaling factors to the array of pressure data.

15. A computer program product comprising machine executable instructions (130), wherein execution of the machine executable instructions causes a computational system to perform a method according to claim 14.
